# EUROPEAN PATENT APPLICATION

(11) **EP 4 706 595 A1**
(43) Date of publication of application: **11.03.2026**
(21) Application number: 24814321.6
(22) Date of filing: 24.05.2024
(51) Int. Cl.: A61F 2/24

(54) **POLYMERIC HEART VALVE AND PREPARATION METHOD THEREFOR**

(30) Priority: 31.05.2023 CN 202310630756
(71) Applicant: Suzhou Hearthill Medical Technology Co., Ltd., Suzhou, Jiangsu 215000 (CN)
(72) Inventor: ZHANG, Zhigang, Suzhou, Jiangsu 215000 (CN)
(74) Representative: Dai, Simin
(86) International application number: PCT/CN2024/095085
(87) International publication number: WO 2024/245122

(57) **Abstract**

Provided by the present disclosure are a polymeric heart valve and a preparation method therefor. The polymeric heart valve includes a plurality of valve leaflets and a valve frame. The valve frame is a hollow cylindrical structure and includes a first end and a second end; the first end is provided with a plurality of column peaks arranged at intervals in a circumferential direction, and plurality of column valleys are correspondingly formed. A count of the plurality of valve leaflets is consistent with a count of the plurality of column peaks. The plurality of valve leaflets are arranged in a center of the first end. The plurality of valve leaflets are sequentially connected to two adjacent column peaks in the circumferential direction. The polymeric heart valve further includes an insert, the insert is a hollow columnar structure matching the valve frame, and the insert includes a third end and a fourth end corresponding to the first end and the second end, respectively. The insert is suitable for being embedded in the valve frame in an axial direction. The plurality of valve leaflets and the valve frame are subjected to one-time injection molding, and the insert is embedded into the valve frame to become a whole when the valve frame and the plurality of valve leaflets are subjected to injection molding. According to the polymeric heart valve provided by the present disclosure, the radial supporting force of the valve frame is increased by optimizing the structure and the process, thereby improving valve performance, simplifying the preparation process, and improving the preparation efficiency.

## Description

### CROSS-REFERENCE TO RELATED APPLICATIONS

This application claims priority to Chinese Patent Application No. 202310630756.3, filed on May 31, 2023, entitled "POLYMERIC HEART VALVE AND PREPARATION METHOD THEREFOR", which is incorporated herein by reference in its entirety.

### TECHNICAL FIELD

The present disclosure relates to the field of medical devices, particularly to a polymeric heart valve and a preparation method therefor.

### BACKGROUND

A heart valve is a valve between an atrium and a ventricle or between a ventricle and an artery. Valves play a crucial role in the never-ending blood circulation activity of the heart: acting as gatekeepers, preventing blood from flowing back to the newly left atrium or ventricle. A prosthetic heart valve is a cardiac implant interventional medical device for the treatment of heart valve disease or defect. The artificial heart was first applied in clinical practice in 1960 and has since gone through stages such as mechanical valves, biological tissue valves, and interventional valves. It has become a very important medical device in the field of cardiovascular therapy.

Patent CN 114126822 A discloses a heart valve, in order to improve the radial support force of the valve frame, valve leaflets and a valve frame are made of materials with different hardnesses, and the valve frame and the valve leaflets are bonded together by means of high-temperature injection molding, etc. This method requires two injection moldings, increasing not only the amount of valve fabrication but also the risk of poor bonding between the valve frame and valve leaflets.

### SUMMARY

Embodiments of the present disclosure provide a polymeric heart valve and a preparation method therefor that optimizes valve performance by improving the radial support of the valve frame by means of optimizing the structure and process.

A polymeric heart valve provided by an embodiment of the present disclosure includes a plurality of valve leaflets and a valve frame. The valve frame is a hollow cylindrical structure and includes a first end and a second end. The first end is provided with a plurality of column peaks arranged at intervals in a circumferential direction, and a plurality of column valleys are correspondingly formed. A count of the plurality of valve leaflets is consistent with a count of the plurality of column peaks. The plurality of valve leaflets are arranged in a center of the first end. The plurality of valve leaflets are sequentially connected to two adjacent column peaks in the circumferential direction. The polymeric heart valve further includes an insert. The insert is a hollow cylindrical structure matching the valve frame. The insert includes a third end and a fourth end corresponding to the first end and the second end, respectively. The insert is suitable for being embedded in the valve frame in an axial direction. The insert includes an axial positioning structure and a radial positioning structure. The plurality of valve leaflets and the valve frame are subjected to one-time injection molding, and the insert is embedded into the valve frame to become a whole when the valve frame and the plurality of valve leaflets are subjected to injection molding.

In some embodiments, the axial positioning structure includes positioning peaks arranged at the third end and corresponding to the plurality of column peaks in the circumferential direction. Each of the plurality of positioning peaks is symmetrically provided on both sides with positioning grooves and formed with positioning side peaks at edges of the positioning peak.

In some embodiments, the axial positioning structure further includes a plurality of sets of positioning teeth arranged at intervals. The plurality of sets of positioning teeth are provided at the fourth end. Circumferential positions of the plurality of sets of the positioning teeth correspond to the plurality of column peaks and the plurality of column valleys on a one-to-one basis. Each set of positioning teeth includes two teeth.

In some embodiments, the radial positioning structure includes positioning protrusions arranged on an outer wall of the insert. The positioning protrusions are uniformly arranged on the outer wall of the insert.

In some embodiments, the center of the plurality of sets of valve leaflets is provided with a gas-permeable groove having a diameter of 0.005 mm-0.01 mm.

In some embodiments, the insert is provided with a plurality of lightening holes. The plurality of lightening holes are arranged at intervals in the circumferential direction. The plurality of lightening holes are arranged between column peaks and column valleys that are adjacent.

In some embodiments, the second end is provided with an annular edge.

In some embodiments, the material of the plurality of valve leaflets and the valve frame are made of a polymer material and the insert is made of a hard material.

In accordance with another aspect of the present disclosure, also provided is an injection mold for preparing the valve described above. The injection mold includes a mold cavity and a mold core. An outer wall of the mold core mates with an inner wall of the mold cavity to form an injection cavity. The insert is arranged in the injection cavity of the injection mold when the valve frame and the plurality of valve leaflets are subjected to one-time injection molding. Apexes of positioning protrusions abut the inner wall of the mold cavity, making a distance between an outer wall of the insert and the inner wall of the mold cavity equal to a distance between an inner wall of the insert and the outer wall of the mold core.

In some embodiments, the mold cavity is provided with a plurality of injection holes that are arranged in correspondence to the first end of the valve frame.

In some embodiments, a plurality of the injection holes are provided in correspondence with the a plurality of column peaks on a one-to-one basis.

In accordance with another aspect of the present disclosure, a preparation method for the valve described above is also provided. The preparation method includes: placing the insert into the mold cavity of the injection mold of the polymeric heart valve, molding the mold core with the mold cavity to form the injection cavity containing the insert; and injecting a polymer material in a molten state into the injection cavity, waiting for the polymer material in the injection cavity to cool to below a glass transition temperature, opening the injection mold to obtain a one-time molded valve containing the insert.

In some embodiments, the preparation method includes: heating the injection mold to 80-100 °C, heating the polymer material to the molten state of 280-300 °C, and injecting into the injection cavity of the injection mold at a rate of 0.3-1 cm³/s under an injection pressure of 50 MPa;
when the polymer material is filled to 85%-98% of the injection cavity, changing the injection pressure to 30 MPa and maintaining for 3-6 s until the filling is completed;
cooling the injection mold to 30-50 °C at a cooling rate of 15-20 °C/min such that the polymer material cools below the glass transition temperature, the glass transition temperature being 85 °C; and
opening the injection mold and removing the one-time molded valve containing the insert from the injection mold.

In some embodiments, the insert includes an axial positioning structure and a radial positioning structure. The axial positioning structure includes positioning peaks arranged at the third end and corresponding to the plurality of column peaks in the circumferential direction. Each of the positioning peaks is symmetrically provided on both sides with positioning grooves and formed with positioning side peaks at edges of the positioning peak. The axial positioning structure further includes a plurality of sets of positioning teeth arranged at intervals. The plurality of sets of positioning teeth are provided at the fourth end. Circumferential positions of the plurality of sets of the positioning teeth correspond to the plurality of column peaks and the column valleys on a one-to-one basis. Each set of positioning teeth includes two teeth. The radial positioning structure includes positioning protrusions provided on the outer wall of the insert. The apexes of the positioning protrusions abut to an inner wall of the mold cavity. The preparation method further includes: placing the insert in the mold cavity according to axial positioning of the positioning peaks, the positioning side peaks, and the plurality of sets of positioning teeth, and radial positioning of the positioning protrusions.

In some embodiments, the cavity is provided with a plurality of injection holes. The plurality of injection holes are arranged in correspondence to the first end of the valve frame. The plurality of the injection holes are provided in correspondence with the plurality of column peaks on a one-to-one basis. The preparation method further includes: injecting the polymer material in the molten state into the injection cavity from the injection holes.

Compared with the prior art, the technical solution of the embodiments of the present disclosure has beneficial effects.

For example, the insert is embedded in the valve frame to improve a radial support force of the valve frame; one-time injection molding of the valve frame and valve leaflets is adopted to optimize the valve performance, avoid using different materials for the valve frame and valve leaflets, resulting in poor bonding between the valve frame and valve leaflets, avoiding two-time injection of the valve frame and valve leaflets, so that the preparation process is simplified and the preparation efficiency is improved.

As another example, by setting positioning structures on the insert, a displacement of the insert caused by an injection pressure during injection molding can be avoided. At the same time, thicknesses from the inner and outer walls of the valve frame to the insert can be uniform, resulting in better wrapping performance.

For a further example, the injection holes are provided on the valve frame to avoid the influence on the valve leaflets by a fracture trace generated when the polymer material injected into a channel is separated from the valve leaflets.

### BRIEF DESCRIPTION OF THE DRAWINGS

FIG. 1 is a structural schematic diagram of a polymeric heart valve in an embodiment of the present disclosure;
FIG. 2 is a top view of a polymeric heart valve according to an embodiment of the present disclosure;
FIG. 3 is a structural schematic diagram of an insert in an embodiment of the present disclosure;
FIG. 4 is a structural schematic diagram of an insert from another view in an embodiment of the present disclosure;
FIG. 5 is a cross-sectional view of AA in FIG. 2;
FIG. 6 is a top view of an injection mold in an embodiment of the present disclosure;
FIG. 7 is a cross-sectional view of AA in FIG. 6;
FIG. 8 is an enlarged partial view of point A in FIG. 7; and
FIG. 9 is a layout diagram of injection holes of a polymeric heart valve in an embodiment of the present disclosure.

### Description of reference numerals:

11, valve frame; 111, first end; 112, second end; 113, column peak; 114, column valley;
21, valve leaflet;
31, insert; 311 third end, 312, fourth end; 313, lightening hole; 314, positioning peak; 315, positioning groove; 316, positioning tooth; 3161, tooth; 317, positioning protrusion; 318, positioning side peak;
41, annular edge;
51, mold cavity; 511, injection hole;
61, mold core; and
71, injection cavity.

### DETAILED DESCRIPTION

In order to make the purpose, features, and beneficial effects of the present disclosure more obvious and understandable, the detailed embodiments of the present disclosure will be described in detail below in conjunction with the accompanying drawings. It can be understood that the specific embodiments described below are only used to explain the present disclosure, and not to limit the present disclosure. Also, the same or similar reference numerals may be used in the drawings to refer to the same or similar elements in different embodiments, and descriptions of the same or similar elements in different embodiments and descriptions of elements, features, effects, etc. of the prior art may be omitted. It should be noted that the axial, radial, and circumferential directions described for the embodiments of the present disclosure refer to the axial, radial, and circumferential directions of the valve frame 11, respectively.

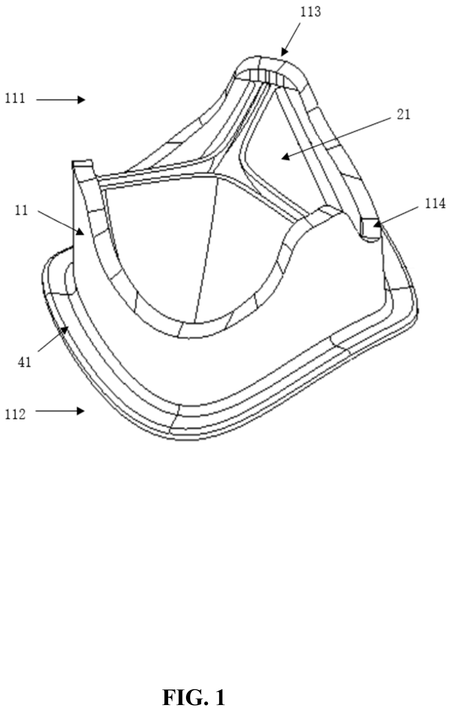

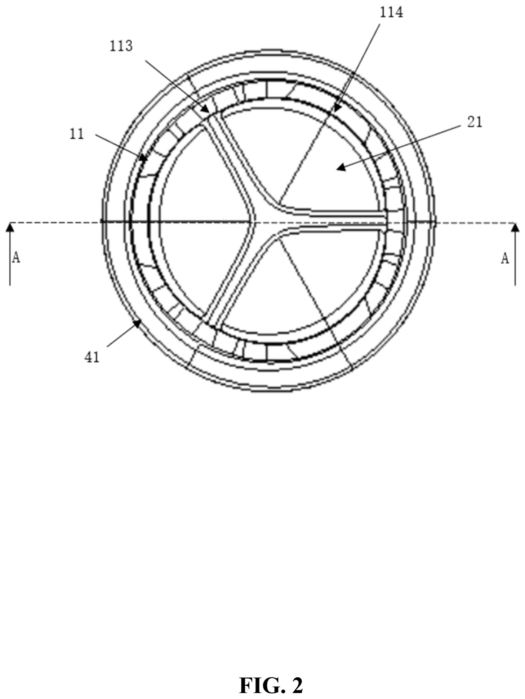

Referring to FIGs. 1-5, embodiments of the present disclosure provide a polymeric heart valve (hereinafter referred to as the valve) .

In some embodiments, a polymeric heart valve includes a plurality of valve leaflets 21 and a valve frame 11. The valve frame 11 is a hollow cylindrical structure and includes a first end 111 and a second end 112. The first end 111 is provided with a plurality of column peaks 113 arranged at intervals in a circumferential direction, and a plurality of column valleys 114 are correspondingly formed. A count of the plurality of valve leaflets 21 is consistent with a count of the plurality of column peaks 113. The plurality of valve leaflets 21 are arranged in a center of the first end 111. The plurality of the valve leaflets 21 sequentially connected to two adjacent column peaks 113 in the circumferential direction. The valve further includes an insert 31. The insert 31 is a hollow cylindrical structure matching the valve frame 11. The insert 31 includes a third end 311 and a fourth end 312 corresponding to the first end 111 and the second end 112, respectively. The insert 31 is suitable for being embedded in the valve frame 11 in an axial direction. The plurality of valve leaflets 21 and the valve frame 11 are subjected to one-time injection molding. The insert 31 is embedded into the valve frame 11 to become a whole when the valve frame 11 and the plurality of valve leaflets 21 are subjected to injection molding.

In some embodiments, the count of the valve leaflets 21 and the count of the column peaks 113 are both three. When the blood flows from the first end 111 to the second end 112, the valve leaflets 21 are closed to prevent the blood from flowing. As blood passes from the second end 112 to the first end 111, the valve leaflets 21 are open to allow blood to pass therethrough.

In some embodiments, the count of the valve leaflets 21 and the count of column peaks 113 are both two.

In some embodiments, the count of the valve leaflets 21 may be selected based on an application position.

In some embodiments, the second end 112 is provided with an annular edge 41.

In some embodiments, the plurality of valve leaflets 21 and the valve frames 11 are made of a polymer material and the insert 31 is made of a hard material. The hard material refers to a composite material, a non-metallic material, or hard alloy with a surface hardness reaching HRC22. The valve frame 11 completely wraps insert 31 so that insert 31 may be metallic or non-metallic.

Since the valve frame 11 and valve leaflets 21 of the polymer material are relatively soft and a radial support force of the entire valve is insufficient, it is necessary to implant an insert 31 within the valve frame 11 to increase the radial support force. The insert 31 needs to withstand a pressure exerted on it by the polymer material during injection molding, withstand a high temperature exerted on it by molten polymers, and meet biocompatibility requirements for long-term implantation in the human body.

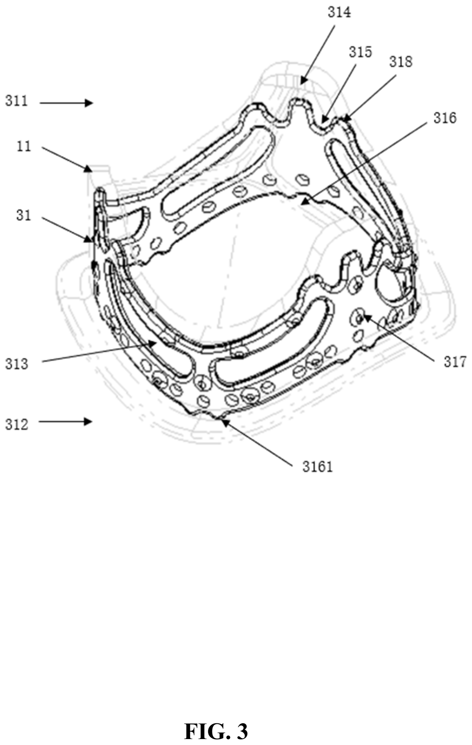

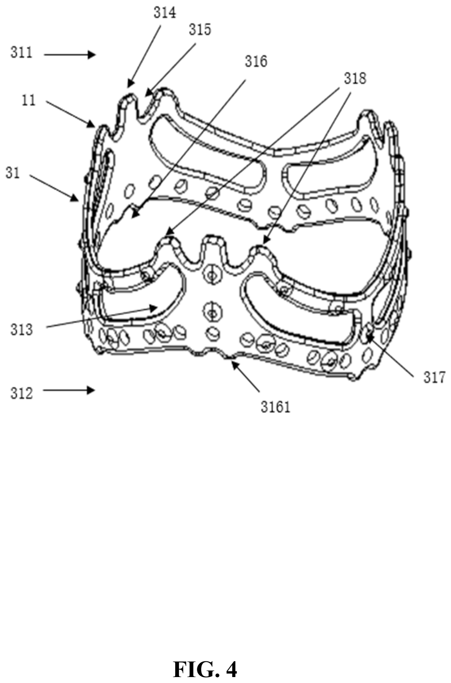

Referring to FIGs. 3 and 4, in some embodiments, the insert 31 includes an axial positioning structure and a radial positioning structure 317. The axial positioning structure includes positioning peaks 314, wherein the positioning peaks 314 are arranged at the third end 311 and are at the same positions as the column peaks 113 in the circumferential direction. Each of the positioning peaks 314 is symmetrically provided on both sides with positioning grooves 315 and formed with positioning side peaks 318 at edges of the positioning peak 314. The positioning side peak 318 realizes the axial and circumferential positioning of the insert 31, so as to avoid an axial displacement or a circumferential displacement of the insert 31 during injection molding due to an injection pressure. The positioning groove 315 increases a wrapping area around the insert 31, realizing a better wrapping effect, so as to avoid the insert 31 separating from the valve frame 11. At the same time, the use of the material of the insert 31 is reduced, saving raw materials.

In some embodiments, the axial positioning structure further includes a plurality of sets of positioning teeth 316 arranged at intervals. The plurality of sets of positioning teeth 316 are provided at the fourth end 312. Circumferential positions of the plurality of sets of the positioning teeth 316 are the same as positions of the column peaks 113 and the plurality of column valleys 114. Each set of positioning teeth 316 includes two teeth 3161. The positioning teeth 316 mate with positioning side peaks 318 for further accurate positioning.

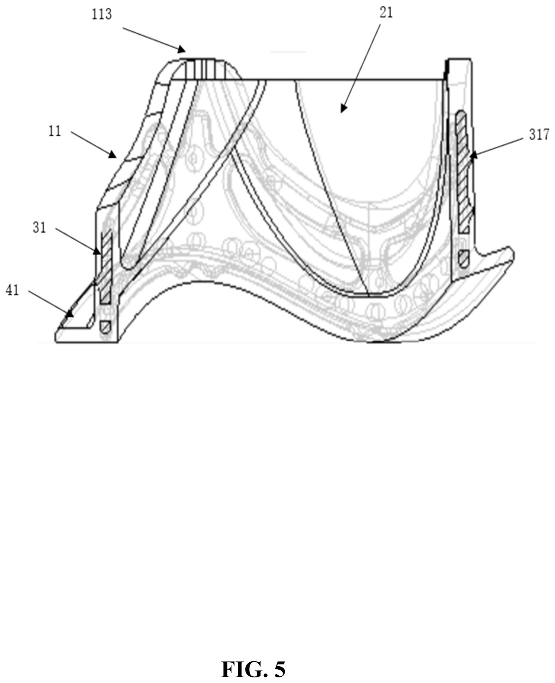

Referring to FIG. 5, in some embodiments, the radial positioning structure 317 includes positioning protrusions 317 arranged on an outer wall of the insert 31. The positioning protrusions 317 are uniformly arranged on the outer wall of the insert 31. The positioning protrusion 317 provides radial positioning of the insert 31, preventing a radial displacement of the insert 31 during injection molding due to the injection pressure.

In some embodiments, the positioning protrusion 317 may be provided in a circular shape or other shapes that facilitate fabrication.

In some embodiments, the insert 31 is provided with a plurality of lightening holes 313. The plurality of lightening holes 313 are arranged at intervals in the circumferential direction. The plurality of lightening holes (313) are arranged between column peaks (113) and column valleys (114) that are adjacent. Without affecting the supporting force of the insert 31, the use of the material of the insert 31 is minimized to save raw materials. At the same time, the wrapping area around the insert 31 is increased, so that the wrapping effect is better and the insert 31 is prevented from separating from the valve frame 11.

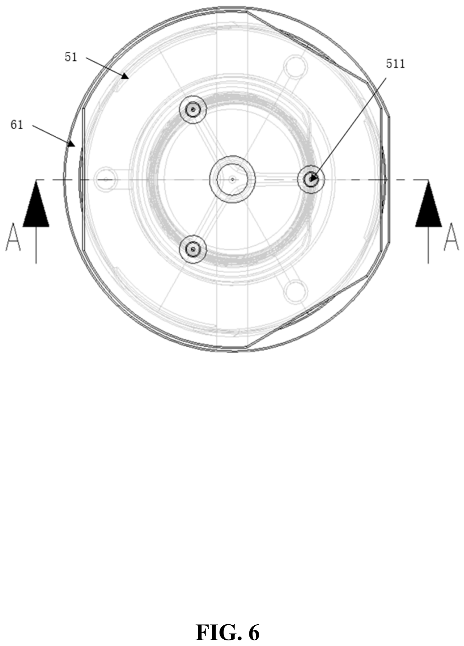

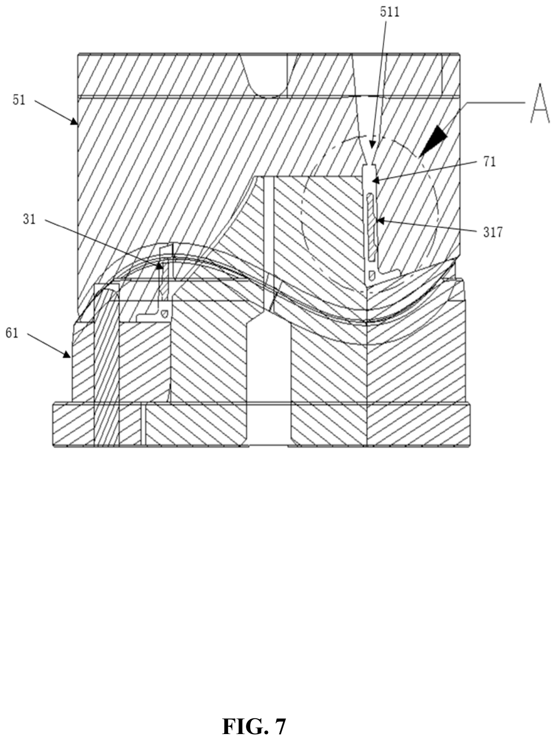

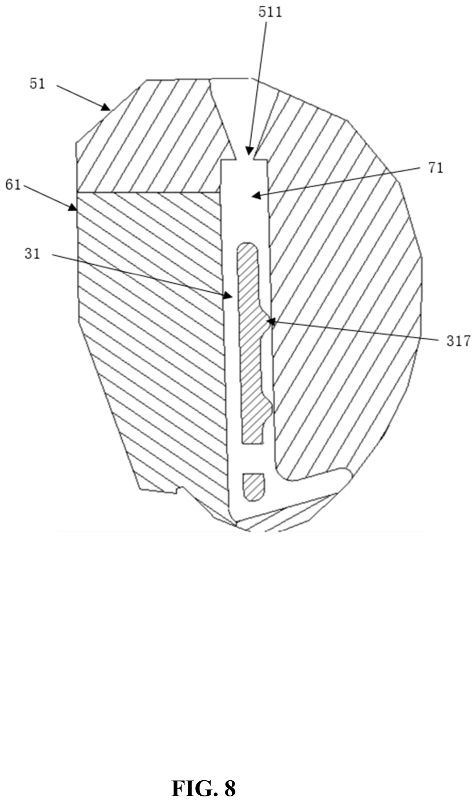

With reference to FIGs. 6-8, in some embodiments, when the valve frame 11 and the plurality of valve leaflets 21 are subjected to one-time injection molding, the insert 31 is arranged in an injection cavity 71 formed by an injection mold. The injection mold includes a mold cavity 51 and a mold core 61. An outer wall of the mold core 61 mates with an inner wall of the mold cavity 51 to form the injection cavity 71. Apexes of the positioning protrusions 317 abut to the inner wall of the mold cavity 51 so that a distance between the outer wall of the insert 31 and the inner wall of the mold cavity 51 is equal to a distance between an inner wall of the insert 31 and the outer wall of the mold core 61. That is to say, thicknesses from the inner wall and the outer wall of the valve frame 11 to the insert 31 are uniform, and the wrapping performance is better, and the insert 31 is not easily dislodged from the valve frame 11.

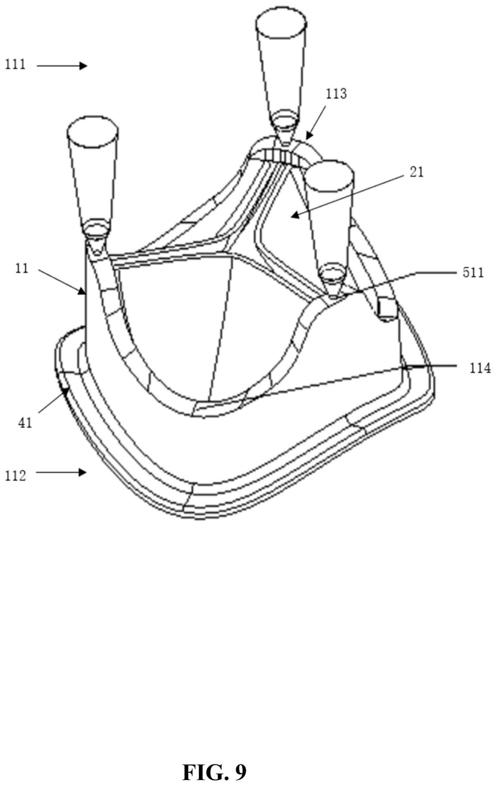

Referring also to FIG. 9, in some embodiments, the mold cavity 51 is provided with injection holes 511 that are arranged in correspondence with an end surface of the first end 111 of the valve frame 11. At the same time, due to the greater thickness of the polymer material at the valve frame 11, it is not easy to form a fracture trace on the surface of the valve frame 11 when the polymer material at the injection hole 511 breaks, and the valve frame 11 plays a supporting role and does not move when in use so that the fracture trace has less effect on the valve frame 11.In the prior art, it is easy to form a fracture trace by arranging the injection holes 511 at a center of the valve leaflets 21, and it is easy to form a crack at the fracture trace due to the opening and closing of the valve leaflets 21 when in use. Compared with the prior art, the valve performance can be optimized by changing the position of the injection holes 511.

In some embodiments, a plurality of injection holes 511 are provided, and the plurality of the injection holes 511 are provided in correspondence with the plurality of column peaks 113 on a one-to-one basis. The plurality of column peaks 113 of the valve frame 11 have the greatest thickness and highest position of polymer material, which facilitates the flow of polymer material during injection filling. In some embodiments, the injection holes 511 may also be provided at the plurality of column valleys 114.

In some embodiments, the center of the valve leaflets 21 is provided with a gas-permeable groove. The gas-permeable groove in the center of the valve leaflets 21 is more conducive to the flow of polymer material at the valve leaflet 21 during injection molding. A diameter of the air-permeable groove is 0.005 mm-0.01 mm. When the diameter is greater than 0.01 mm, the polymer material may overflow, forming a rough edge on the surface of the valve leaflets 21. When the diameter is less than 0.005 mm, venting may be hindered, which affects the flow of the polymer material.

In order to facilitate an understanding of the technical solution of the polymeric heart valve provided in the embodiments of the present disclosure, a preparation method for the heart valve is described.

In some embodiments, the preparation method may include the following steps.
S1: placing the insert 31 in the mold cavity 51 according to axial and circumferential positioning of the positioning peaks 314 and the plurality of sets of positioning teeth 316 and radial positioning of the positioning protrusions 317;
S2: molding the mold core 61 with the mold cavity 51 to form the injection cavity 71 containing the insert 31;
S3: heating the injection mold to 80-100 °C, preferably to 90 °C; heating the polymer material to a molten state of 280-300 °C, preferably at a temperature of 290 °C; and injecting the polymer material into the injection cavity 71 of the injection mold at a rate of 0.3-1 cm³/s, preferably at a rate of 0.5 cm³/s, under an injection pressure of 50 MPa;
S4: when the polymer material is filled to 85%-98% of the injection cavity 71, changing the injection pressure to 30 MPa and maintaining for 3-6 s, preferably for 4.5 s, until the filling is completed;
S5: cooling the injection mold to 30-50 °C, preferably 40 °C, at a cooling rate of 15-20 °C/min, such that the polymer material cools below a glass transition temperature, the glass transition temperature being 85 °C;
S6: opening the injection mold and removing a one-time molded valve containing the insert 31 from the injection mold.

While specific embodiments have been described above, these embodiments are not intended to limit the scope of the disclosure, even if a single embodiment is described with respect to only certain features. The examples of features provided in this disclosure are intended to be illustrative, and not limiting, unless expressed differently. In some embodiments, the technical features of one or more dependent claims can be combined with the technical features of independent claims as technically feasible according to actual needs, and the technical features from the corresponding independent claims can be combined in any appropriate way rather than just through specific combinations listed in the claims.

Although the present disclosure is disclosed above, the present disclosure is not limited thereto. Various changes and modifications may be made by one skilled in the art without departing from the spirit and scope of the present disclosure, which is intended to be limited only by the scope of the appended claims.

## Claims

1. A polymeric heart valve, comprising a plurality of valve leaflets (21) and a valve frame (11), wherein the valve frame (11) is a hollow cylindrical structure and comprises a first end (111) and a second end (112); the first end (111) is provided with a plurality of column peaks (113) arranged at intervals in a circumferential direction, and a plurality of column valleys (114) are correspondingly formed; a count of the plurality of valve leaflets (21) is consistent with a count of the plurality of column peaks (113); the plurality of valve leaflets (21) are arranged in a center of the first end (111); the plurality of the valve leaflets (21) are sequentially connected to two adjacent column peaks (113) in the circumferential direction; the polymeric heart valve further comprises an insert (31); the insert (31) is a hollow cylindrical structure matching the valve frame (11); and the insert (31) comprises a third end (311) and a fourth end (312) corresponding to the first end (111) and the second end (112), respectively; the insert (31) is suitable for being embedded in the valve frame (11) in an axial direction; the insert (31) comprises an axial positioning structure and a radial positioning structure (317); the plurality of valve leaflets (21) and the valve frame (11) are subjected to one-time injection molding; the insert (31) is embedded into the valve frame (11) to become a whole when the valve frame (11) and the plurality of valve leaflets (21) are subjected to injection molding.

2. The polymeric heart valve according to claim 1, wherein the axial positioning structure comprises positioning peaks (314) arranged at the third end (311) and corresponding to the plurality of column peaks (113) in the circumferential direction; each of the positioning peaks (314) is symmetrically provided on both sides with positioning grooves (315) and formed with positioning side peaks (318) at edges of the positioning peak (314).

3. The polymeric heart valve according to claim 1, wherein the axial positioning structure further comprises a plurality of sets of positioning teeth (316) arranged at intervals; the plurality of sets of positioning teeth (316) are provided at the fourth end (312); circumferential positions of the plurality of sets of the positioning teeth (316) correspond to the plurality of column peaks (113) and the plurality of column valleys (114) on a one-to-one basis; and each set of positioning teeth (316) comprises two teeth (3161).

4. The polymeric heart valve according to claim 1, wherein the radial positioning structure (317) comprises positioning protrusions (317) arranged on an outer wall of the insert (31); and the positioning protrusions (317) are uniformly arranged on the outer wall of the insert (31).

5. The polymeric heart valve according to claim 1, wherein a center of the plurality of valve leaflets (21) is provided with a gas-permeable groove having a diameter of 0.005 mm-0.01 mm.

6. The polymeric heart valve according to claim 1, wherein the insert (31) is provided with a plurality of lightening holes (313); the plurality of lightening holes (313) are arranged at intervals in the circumferential direction; and the plurality of lightening holes (313) are arranged between column peaks (113) and column valleys (114) that are adjacent.

7. The polymeric heart valve according to claim 1, wherein the second end (112) is provided with an annular edge.

8. The polymeric heart valve according to claim 1, wherein the plurality of valve leaflets (21) and the valve frame (11) are made of a polymer material, and the insert (31) is made of a hard material.

9. An injection mold for preparing the polymeric heart valve according to any one of claims 1-8, wherein the injection mold comprises a mold cavity (51) and a mold core (61); an outer wall of the mold core (61) mates with an inner wall of the mold cavity (51) to form an injection cavity (71); the insert (31) is arranged in the injection cavity (71) of the injection mold when the valve frame (11) and the plurality of valve leaflets (21) are subjected to one-time injection molding; and apexes of positioning protrusions (317) abut the inner wall of the mold cavity (51), making a distance between an outer wall of the insert (31) and the inner wall of the mold cavity (51) equal to a distance between an inner wall of the insert (31) and the outer wall of the mold core (61).

10. The injection mold according to claim 9, wherein the mold cavity (51) is provided with a plurality of injection holes (511) that are arranged in correspondence to the first end (111) of the valve frame (11).

11. The injection mold according to claim 9, wherein a plurality of the injection holes (511) are provided in correspondence with the plurality of column peaks (113) on a one-to-one basis.

12. A preparation method for the polymeric heart valve according to any one of claims 1-8, using the injection mold according to any one of claims 9-11, comprising:
placing the insert (31) into the mold cavity (51) of the injection mold of the polymeric heart valve, molding the mold core (61) with the mold cavity (51) to form the injection cavity (71) containing the insert (31); and
injecting a polymer material in a molten state into the injection cavity (71), waiting for the polymer material in the injection cavity (71) to cool to below a glass transition temperature, opening the injection mold to obtain a one-time molded valve containing the insert (31).

13. The preparation method according to claim 12, comprising:
heating the injection mold to 80-100 °C, heating the polymer material to the molten state of 280-300 °C, and injecting the polymer material into the injection cavity (71) of the injection mold at a rate of 0.3-1 cm³/s under an injection pressure of 50 MPa;
when the polymer material is filled to 85%-98% of the injection cavity (71), changing the injection pressure to 30 MPa and maintaining for 3-6 s until the filling is completed;
cooling the injection mold to 30-50 °C at a cooling rate of 15-20 °C/min such that the polymer material cools below the glass transition temperature, the glass transition temperature being 85 °C; and
opening the injection mold and removing the one-time molded valve containing the insert (31) from the injection mold.

14. The preparation method according to claim 12, wherein the axial positioning structure comprises positioning peaks (314) arranged at the third end (311) and corresponding to the plurality of column peaks (113) in the circumferential direction; each of the positioning peaks (314) is symmetrically provided on both sides with positioning grooves (315) and formed with positioning side peaks (318) at edges of the positioning peak (314); the axial positioning structure further comprises a plurality of sets of positioning teeth (316) arranged at intervals; the plurality of sets of positioning teeth (316) are provided at the fourth end (312); circumferential positions of the plurality of sets of the positioning teeth (316) correspond to the plurality of column peaks (113) and the plurality of column valleys (114) on a one-to-one basis; and each set of positioning teeth (316) comprises two teeth (3161); the preparation method further comprises:
placing the insert (31) in the mold cavity (51) according to axial positioning of the positioning peaks (314), the positioning side peaks (318) and the plurality of sets of positioning teeth (316) and radial positioning of the positioning protrusions (317).

15. The preparation method according to claim 13, wherein the mold cavity (51) is provided with a plurality of injection holes (511) that are arranged in correspondence to the first end (111) of the valve frame (11); and the plurality of injection holes (511) are provided in correspondence with the plurality of column peaks (113) on a one-to-one basis; and the preparation method further comprises:
injecting the polymer material in the molten state into the injection cavity (71) from the plurality of injection holes.
